(19) Europäisches Patentamt
European Patent Office
Office européen des brevets

(11) Publication number: **0 503 508 A1**

# (12) EUROPEAN PATENT APPLICATION

(21) Application number: **92103902.0**

(51) Int. Cl.[5]: **A61K 9/127**

(22) Date of filing: **06.03.92**

(30) Priority: **13.03.91 JP 48079/91**

(43) Date of publication of application: **16.09.92 Bulletin 92/38**

(84) Designated Contracting States: **DE FR GB**

(71) Applicant: **Kao Corporation**
**1-14-10, Nihonbashi Kayaba-cho**
**Chuo-ku Tokyo(JP)**

(72) Inventor: **Masuda, Mitsuharu**
**2-9-1-302, Yamate**
**Funabashi-shi, Chiba(JP)**
Inventor: **Kurosaki, Tomihiro**
**1465 Tannowa, Misaki-cho**
**Sennan-gun, Osaka-fu(JP)**
Inventor: **Yano, Shinji**
**446-43, Oaza-Nakaguro, Iwade-cho**
**Naga-gun, Wakayama(JP)**

(74) Representative: **Wächtershäuser, Günter, Dr.**
**Tal 29**
**W-8000 München 2(DE)**

## (54) Vesicles and polymeric vesicles.

(57) A vesicle comprising, as a membrane-forming ingredient, a phosphoric ester represented by the following general formula (2):

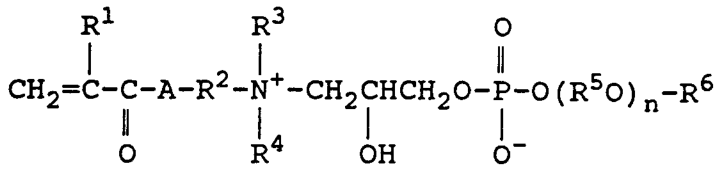

(2)

wherein $R^1$ means a hydrogen atom or an alkyl group having 1-4 carbon atoms, $R^2$ denotes an alkylene group having 1-6 carbon atoms, $R^3$ and $R^4$ may be identical to or different from each other and represent individually an alkyl group having 1-4 carbon atoms, $R^5$ stands for an alkylene group having 2-3 carbon atoms, $R^6$ means a linear or branched alkyl or alkenyl group which may be substituted by halogen atoms and has 8-36 carbon atoms, a phenyl group which has been substituted by a linear or branched alkyl group having 3-15 carbon atoms or a group represented by the following general formula (3) or (4):

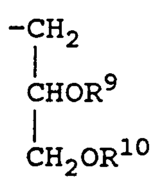

(3)

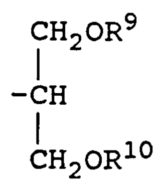

(4)

in which $R^9$ and $R^{10}$ may be identical to or different from each other and mean individually a hydrogen atom, a

EP 0 503 508 A1

saturated or unsaturated, linear or branched acyl group having 5-36 carbon atoms or a linear or branched alkyl or alkenyl group having 5-36 carbon atoms, provided that both $R^9$ and $R^{10}$ are not a hydrogen atom at the same time, A denotes NH or an oxygen atom, and n stands for a number of from 0-30, and a polymeric vesicle comprising, as a membrane-forming ingredient, a polymer of the phosphoric ester represented by the general formula (2).

## BACKGROUND OF THE INVENTION

### Field of the Invention:

This invention relates to a novel vesicle or polymeric vesicle, and more specifically to a vesicle or polymeric vesicle which is stable, can be formed in various particle sizes, can contain various effective ingredients therein and is easy to produce in industry.

### Discussion of the Background:

A cell is the smallest structural unit of the living body. The cell membrane performs a variety of biologically important functions such as partitioning between adjacent cells and compartmentation, cell movement, material transport, and information transfer.

Recently in the field of polymer chemistry, extensive work has been done to prepare an artificial polymeric membrane having those functions inherent in cell membranes. Such an artificial polymeric membrane would be applicable to a wide variety of fields such as engineering, medical science and pharmacy. For example, it has been confirmed that the cell membrane is composed of a phospholipid. Due to the amphipathic nature of phospholipid (having both hydrophobic and hydrophilic groups in the same molecule), it tends to organize itself in an orderly fashion to form a tissue having a bimolecular layer structure. Therefore, vesicles which have a bimolecular layer similar to a cell membrane have been prepared from a natural phospholipid to study their application as a model of a biomembrane or microcapsule.

In the medical field, vesicles are utilized as a carrier for a drug as it is encapsulated in them. By the use of vesicles, the drug can reach the target organ without being deactivated or interfered. Any advance side effects of drugs can also be mitigated by encapsulating substances having affinity with the target organ in vesicles and administered. Furthermore, vesicles are utilized in the fields of immuno-assays, cosmetic compositions, various micro capsules and release-controlled perfumes.

Alternatively, in recent years, various kinds of non-phospholipid amphipathic compounds have been synthesized which have a similar tendency to self-organize and form a bimolecular layer structure, permitting the formation of vesicles. Furthermore, polymeric vesicles have been studied extensively in which the individual units forming the bimolecular layer structure have been polymerized to stabilize the membrane. Compounds usable as monomers are those whose hydrophobic or hydrophilic group have a polymerizable group. An example of such a compound, represented by the following formula (1), has been synthesized by Regen et al. [J. Am. Chem. Soc., 105, 2975 (1983)].

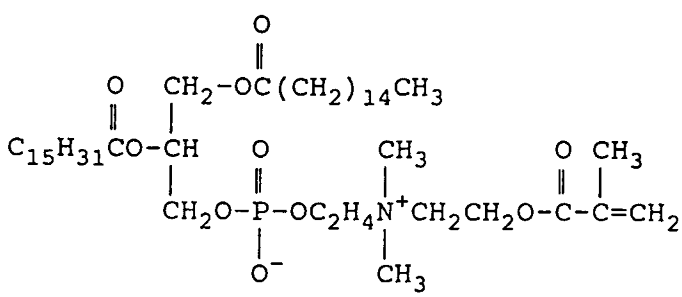

(1)

It has been desired to develop a monomer, for use in preparing polymeric vesicles, having physical characteristics such as the ability to impart surface activity and the tendency to self-organize in addition to the inherent chemical properties of the monomer, and being compatible with the living body, in view of the future application of the vesicles to medicines, medical polymers, etc.

Phospholipids surely have these desirable characteristics, including the ability to impart surface activity, the tendency to self-organize, the biocompatibility and the safety. Keen attention has been paid to compounds obtained by introducing a polymerizable group in phospholipids. However, the synthesis of phosphoric esters and phospholipid analogues which contain a polymerizable group requires complicated reaction schemes and is often tremendously difficult to perform industrially. Accordingly, it is very difficult to industrially produce vesicles by using these phosphoric esters and phospholipid analogues as membrane-forming ingredients.

There has therefore been a demand for the development of a vesicle which is easy to produce in

industry and widely applicable to various functional materials, microcapsules and the like in fields of engineering, medical science, etc.

## SUMMARY OF THE INVENTION

Accordingly, one object of this invention is to provide a vesicle having wide applicability in a variety of fields such as engineering, medical science and pharmacy, and which is easy to produce industrially.

A further object of this invention is to provide a polymeric vesicle wherein the structural units forming the membrane have been polymerized and which is widely applicable to various fields such as medicine, engineering and pharmacy, and is easy to produce industrially.

A still further object of this invention is to provide a synthetic vesicle capable of encapsulating a physiologically active substance.

A further object of this invention is to provide a synthetic polymeric vesicle capable of encapsulating a physiologically active substance.

Still a further object of this invention is to provide a compound usable as a membrane forming ingredient in the preparation of the desired vesicles or polymeric vesicles.

These and other objects, features and attendant advantages of the present invention which will be more fully appreciated as the same becomes better understood from the following detailed description, have been achieved by the discovery that a phosphoric ester represented by the following general formula (2):

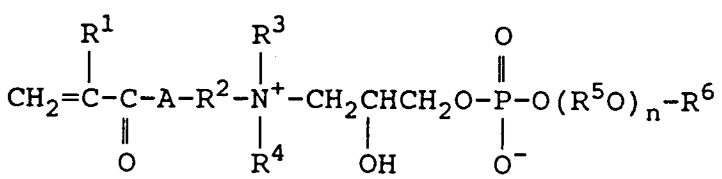

(2)

wherein $R^1$ means a hydrogen atom or an alkyl group having 1-4 carbon atoms, $R^2$ denotes an alkylene group having 1-6 carbon atoms, $R^3$ and $R^4$ may be identical to or different from each other and represent individually an alkyl group having 1-4 carbon atoms, $R^5$ stands for an alkylene group having 2-3 carbon atoms, $R^6$ means a linear or branched alkyl or alkenyl group which may be substituted by halogen atoms and has 8-36 carbon atoms, a phenyl group which has been substituted by a linear or branched alkyl group having 3-15 carbon atoms or a group represented by the following general formula (3) or (4):

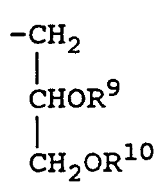

(3)

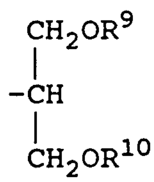

(4)

in which $R^9$ and $R^{10}$ may be identical to or different from each other and mean individually a hydrogen atom, a saturated or unsaturated, linear or branched acyl group having 5-36 carbon atoms or a linear or branched alkyl or alkenyl group having 5-36 carbon atoms, provided that both $R^9$ and $R^{10}$ are not a hydrogen atom at the same time, A denotes NH or an oxygen atom, and n stands for a number of from 0-30;

can be used, with or without polymerization, as a membrane-forming ingredient in the production of a vesicle or polymeric vesicle, respectively.

## DETAILED DESCRIPTION OF THE PREFERRED EMBODIMENTS

The vesicle of the present invention comprises, as a membrane-forming ingredient, a phosphoric ester represented by the following general formula (2):

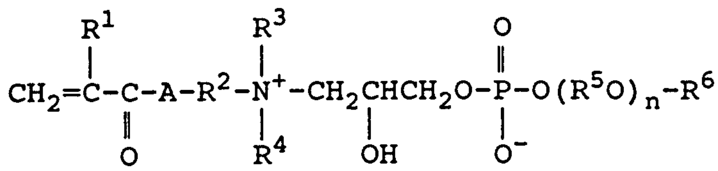

(2)

wherein $R^1$ means a hydrogen atom or an alkyl group having 1-4 carbon atoms, $R^2$ denotes an alkylene group having 1-6 carbon atoms, $R^3$ and $R^4$ may be identical to or different from each other and represent individually an alkyl group having 1-4 carbon atoms, $R^5$ stands for an alkylene group having 2-3 carbon atoms, $R^6$ means a linear or branched alkyl or alkenyl group which may be substituted by halogen atoms and has 8-36 carbon atoms, a phenyl group which has been substituted by a linear or branched alkyl group having 3-15 carbon atoms or a group represented by the following general formula (3) or (4):

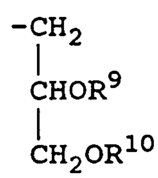

(3)

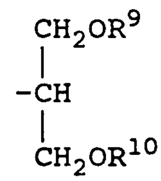

(4)

in which $R^9$ and $R^{10}$ may be identical to or different from each other and mean individually a hydrogen atom, a saturated or unsaturated, linear or branched acyl group having 5-36 carbon atoms or a linear or branched alkyl or alkenyl group having 5-36 carbon atoms, provided that both $R^9$ and $R^{10}$ are not a hydrogen atom at the same time, A denotes NH or an oxygen atom, and n stands for a number of from 0-30, can be used, with or without polymerization, as a membrane-forming ingredient in the production of a vesicle or polymeric vesicle, respectively.

In the phosphoric ester represented by the general formula (2) according to the present invention, the alkyl groups designated by $R^1$, $R^3$ and $R^4$ and having 1-4 carbon atoms include methyl, ethyl, propyl, isopropyl, butyl, isobutyl and tert-butyl.

As examples of the alkylene group indicated by $R^2$ and having 1-6 carbon atoms, may be mentioned methylene, ethylene, propylene, trimethylene, tetramethylene, pentamethylene and hexamethylene groups. The alkylene group designated by $R^5$ and having 2-3 carbon atoms includes ethylene, propylene and trimethylene groups.

As examples of the linear or branched alkyl or alkenyl group, which is indicated by $R^6$, may be substituted by halogen atoms and has 8-36 carbon atoms, may be mentioned octyl, decyl, dodecyl, tetradecyl, hexadecyl, octadecyl, eicosyl, docosyl, tetracosyl, hexacosyl, octacosyl, triacontyl, dotriacontyl, tetratriacontyl, hexatriacontyl, 2-ethylhexyl, 2-butyloctyl, 2-hexyldecyl, 2-octyldodecyl, 2-decyltetradecyl, 2-dodecylhexadecyl, 2-tetradecyloctadecyl, 2-hexadecyleicosyl, monomethyl-branched isostearyl, octenyl, decenyl, dodecenyl, tetradecenyl, hexadecenyl, octadecenyl, eicocenyl, dococenyl, tetracocenyl, hexacocenyl, octacocenyl, triacontenyl, dotriacontenyl, tetratriacontenyl, hexatriacontenyl, tridecafluorooctyl, heptadecafluorodecyl, heneicosafluorododecyl, pentacosafluorotetradecyl, nonacosafluorohexadecyl, tritriacontafluorooctadecyl, 2-pentafluoroethylpentafluorohexyl, 2-tridecafluorohexyltridecafluorodecyl, 2-heptadecafluoro-octylheptadecafluorododecyl, 2-heneicosafluorodecylheneicosafluorotetradecyl, 2-pentacosafluorododecylpentacosafluorohexadecyl and 2-nonacosafluorotetradecylnonacosafluorooctadecyl groups. As examples of the phenyl group substituted by a linear or branched alkyl group having 3-15 carbon atoms, may be mentioned propylphenyl, butylphenyl, hexylphenyl, octylphenyl, nonylphenyl, decylphenyl and dodecylphenyl groups.

As examples of the saturated or unsaturated, linear or branched acyl groups having 5-36 carbon atoms and linear or branched alkyl or alkenyl group having 5-36 carbon atoms, which are indicated by $R^9$ and $R^{10}$, may be mentioned valeryl, hexanoyl, octanoyl, decanoyl, dodecanoyl, tetradecanoyl, hexadecanoyl, octadecanoyl, eicosanoyl, docosanoyl, tetracosanoyl, hexacosanoyl, octacosanoyl, triacontanoyl, dotriacontanoyl, tetratriacontanoyl, hexatriacontanoyl, dodecenoyl, tetradecenoyl, hexadecenoyl, oleoyl, eicocenoyl, dococenoyl, tetracocenoyl, hexacocenoyl, octacocenoyl, triacontenoyl, dotriacontenoyl, tetratriacontenoyl,

hexadienoyl, octadecadienoyl, eicosadienoyl, docosadienoyl, tetracosadienoyl, hexacasadienoyl, octacosadienoyl, triacontadienoyl, dotriacontadienoyl, tetratriacontadienoyl, hexatriacontadienoyl, hexadecatrienoyl, octadecatrienoyl, eicosatrienoyl, docosatrienoyl, hexadecatetraenoyl, octadecatetraenoyl, eicosatetraenoyl, docosatetraenoyl, eicosapentaenoyl, docosapentaenoyl, tetracosahexaenoyl, octadecanoyl, pentyl, hexyl, octyl, decyl, dodecyl, tetradecyl, hexadecyl, octadecyl, eicosyl, docosyl, tetracosyl, hexacosyl, octacosyl, triacontyl, dotriacontyl, tetratriacontyl, hexatriacontyl, 2-ethylhexyl, 2-butyloctyl, 2-hexyldecyl, 2-octyldodecyl, 2-decyltetradecyl, 2-dodecylhexadecyl, 2-tetradecyl-octadecyl, 2-hexadecyleicosyl, monomethyl-branched isostearyl, octenyl, decenyl, dodecenyl, tetradecenyl, hexadecenyl, octadecenyl, eicocenyl, dococenyl, tetracocenyl, hexacocenyl, octacocenyl, triacontenyl, dotriacontenyl, tetratriacontenyl and hexatriacontenyl groups.

Among these groups represented by $R^6$, branched alkyl or alkenyl groups which may be substituted by halogen atoms and have 8-36 carbon atoms are preferred.

Such branched alkyl or alkenyl groups include those having a branched site at $\alpha$, $\beta$, or any site. As examples of the alkyl groups having $\beta$ branched site, may be mentioned preferably 2-ethylhexyl, 2-ethylhexadecyl, 2-butyltetradecyl, 2-hexyldecyl, 2-butylhexadecyl, 2-octyldodecyl, 2-decyltetradecyl, 2-tetradecyloctadecyl, 2-ethyloctadecyl, 2-dodecylhexadecyl, 2-hexadecyleicosyl. Among these groups, the groups having 10-28 carbon atoms, for example, 2-hexyldecyl, 2-octyldecyl and 2-decyltetradecyl are especially preferred.

As examples of the phosphoric ester (2), wherein the groups represented by $R^1$ to $R^6$ are as described above, may be mentioned 2-hexyldecyl 2-hydroxy-3-[N,N-dimethyl-N-(3-methacrylamidopropyl) ammonio]-propyl phosphate, 2-decyltetradecyl 2-hydroxy-3-[N,N-dimethyl-N-(3-methacrylamidopropyl)ammonio]propyl phosphate and 2-tetradecyl-octadecyl 2-hydroxy-3-[N-methyl-N-ethyl-N-(3-acrylamidopropyl)ammonio]-propyl phosphate. Such examples may also include the phosphoric ester (2) wherein the group represented by $R^6$ are dipalmitoylphosphatidyl, alkyl group originated from an egg yolk phospholipid and alkyl group originated from a soybean phospholipid.

No particular limitation is imposed on the preparation process of the phosphoric ester (2) described above. The phosphoric ester (2) may therefore be prepared by any known processes. For example, it may be obtained by reacting a phosphoric ester (6) or (7) to an amine compound (5) in accordance with the following reaction formula (A) or (B) as disclosed in Japanese Patent Application Laid-Open No. 249994/1987.

Reaction formula (A):

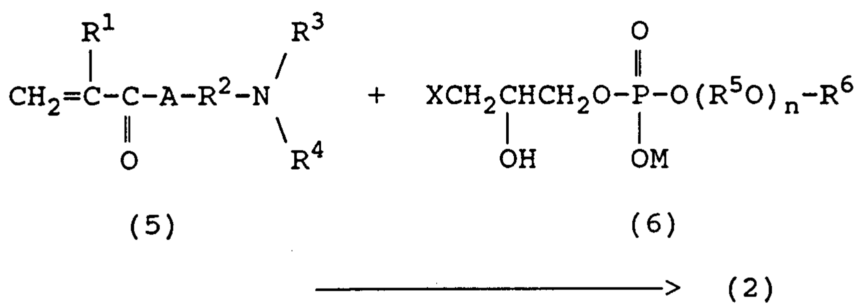

(5) (6) (2)

wherein X means a halogen atom, M denotes an alkali metal, and $R^1$, $R^2$, $R^3$, $R^4$, $R^5$, $R^6$, A and n have individually the meaning defined above.

Reaction formula (B):

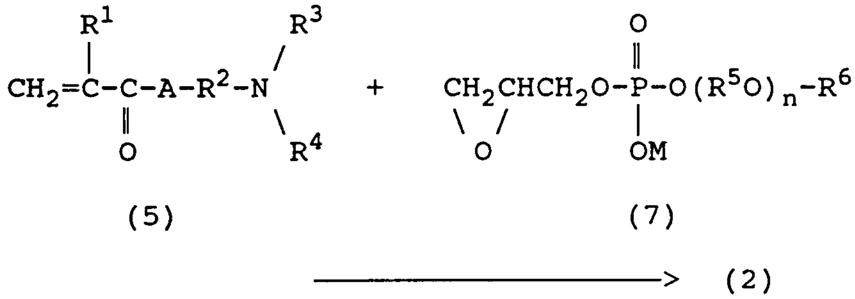

(5) (7) (2)

wherein M, $R^1$, $R^2$, $R^3$, $R^4$, $R^5$, $R^6$, A and n have individually the meaning defined above.

The vesicle according to this invention may contain, as membrane-forming ingredients, one or more compounds such as surfactants, unsaturated monomers [other than the phosphoric ester (2) described above], membrane-stabilizing agents and/or antioxidants in addition to the phosphoric ester represented by the general formula (2).

As the surfactant may be used anionic surfactants, cationic surfactants, nonionic surfactants and amphoteric surfactants. The surfactant itself may or may not be polymerizable and moreover it may or may not possess the ability to form a vesicle by itself.

As examples of the anionic surfactants, may be mentioned alkyl sulfates, polyoxyethylene alkyl ether sulfates, alkylbenzene sulfonates, saturated long chain fatty acid salts, unsaturated long chain fatty acid salts, polyunsaturated long chain fatty acid salts, monoalkyl phosphates, dialkyl phosphates, polyoxyethylene alkyl ether phosphates, and phospholipide derivatives such as phosphatidyl glucose, phosphatidyl mannose, phosphatidyl polyethylene glycol, phosphatidyl glycerol, phosphatidyl polyglycerol, phosphatidyl sorbitol and diphosphatidyl polyethylene glycol.

As examples of the cationic surfactants, may be mentioned dialkylammonium salts, trialkylammonium salts and tetraalkylammonium salts.

As examples of the nonionic surfactants, may be mentioned polyoxyethylene alkyl ethers, polyoxyethylene alkyl phenyl ethers, glycerol alkyl ethers, sorbitan fatty acid esters, polyoxyethylene sorbitan fatty acid esters, polyoxyethylene sorbitol fatty acid esters, propylene glycol fatty acid esters, polyglycerol fatty acid esters, glycerol fatty acid esters, polyoxyethylene fatty acid esters, polyoxyethylene alkylamines, long chain alcohols, and saccharic surfactants such as alkyl glycosides, polyalkyl glycosides and sucrose fatty acid esters.

As examples of the amphoteric surfactants, may be mentioned alkylbetaines, phosphobetaines, sulfobetaines, and natural and synthetic phospholipides such as phosphatidyl choline and phosphatidyl ethanolamine and analogues thereof.

As examples of the unsaturated monomer useful in the practice of this invention, may be mentioned oil-soluble ethylenically unsaturated monomers, hydrophilic unsaturated monomers and crosslinkable unsaturated monomers.

As examples of the oil-soluble ethylenically unsaturated monomers, may be mentioned styrene monomers such as styrene, p-methylstyrene and p-chlorostyrene; acrylic ester monomers such as methyl acrylate, ethyl acrylate, 2-ethylhexyl acrylate, dodecyl acrylate, dimethylaminoethyl acrylate and diethylaminoethyl acrylate; methacrylic ester monomers such as methyl methacrylate, ethyl methacrylate, dodecyl methacrylate, dimethylaminoethyl methacrylate and diethylaminoethyl methacrylate; alkyl vinyl ethers such as methyl vinyl ether and ethyl vinyl ether; vinyl ester monomers such as vinyl acetate and vinyl butyrate; N-alkyl-substituted (meth)acrylamide such as N-methylacrylamide, N-ethylacrylamide, N-methylmethacrylamide and N-ethylmethacrylamide; and nitrile monomers such as acrylonitrile and methacrylonitrile.

As examples of the hydrophilic unsaturated monomers, may be mentioned (meth)acrylic ester monomers such as hydroxyethyl (meth)acrylate, diethylene glycol mono(meth)acrylate, polyoxyethylene mono(meth)acrylate, butanediol mono(meth)acrylate, glycerol mono(meth)acrylate and polyethylene glycol polypropylene glycol mono(meth)acrylate; and water-soluble vinyl monomers such as N-vinylpyrrolidone and N-vinyloxazolydone.

As examples of the crosslinkable unsaturated monomers, may be mentioned polyfunctional monomers

such as divinylbenzene, ethylene glycol di(meth)acrylate, diethylene glycol (meth)acrylate, trimethylolpropane tri(meth)acrylate and dipentaerythritol hexa(meth)acrylate.

Further, examples of the membrane-stabilizing agent useful in the practice of this invention include sterols such as cholesterol and cholestanol. An example of the antioxidant would include tocophenol.

Ingredients other than those described above may also be used, either singly or in combination, as membrane-forming ingredients for the vesicles of this invention. If the compound does not form a bimolecular layer by itself, such as cholesterol, it is preferred that the proportion of the compound to be added should be suitably controlled so as not to destroy the vesicular structure formed by the phosphoric ester (2).

In order to obtain the vesicle according to this invention, the phosphoric ester (2) is first mixed with water to give a concentration of 0.01-20 wt.%, preferably, 1-10 wt.%. Further, if desired, a surfactant and/or unsaturated monomer may be incorporated in a proportion of about 0-0.5 mole per mole of phosphoric ester (2). Additionally, if desired, a membrane-stabilizing agent and/or antioxidant may be mixed in a proportion of about 0-0.2 mole per mole of phosphoric ester (2). If the mixture contains an additional unsaturated monomer, the resulting mixture is then stirred while heating at a low temperature, preferably, at a temperature in a range of 10-50°C so as to prevent heat polymerization. The vesicle is thereby formed from the solution with the vesicular membrane-forming ingredient fully dispersed therein. When a membrane-stabilizing agent and/or antioxidant are incorporated, they may be either mixed with the vesicular membrane-forming ingredient in advance or added after formation of the vesicle.

No particular limitation is imposed on the formation of the vesicle. The vesicle may therefore be formed by any suitable method, for example, conventionally-known methods such as ultrasonication, the injection method, the French press method, the dialysis method or the hydration method. The vesicular diameter can be controlled depending on the phosphoric ester (2) used and its mixing ratio with the other membrane-forming ingredients used, the forming method and the forming conditions.

It is also possible to obtain a polymeric vesicle by further carrying out a polymerization between a vesicle comprising, as a membrane-forming ingredient, the phosphoric ester (2) and phosphoric ester (2) itself or an unsaturated monomer other than (2).

In this invention, any suitable method may be used as a polymerization method, and no particular limitation is imposed thereon. It is possible to use the conventionally-known methods, for example, ultraviolet irradiation, $\gamma$ ray irradiation, addition of polymerization initiator, etc.

No particular limitation is imposed on substances which may be encapsulated in the vesicle or polymeric vesicle according to this invention. These may be either hydrophobic substances, hydrophilic substances or mixtures thereof. As exemplary physiologically active substances to be contained in the vesicle or polymeric vesicle, may be mentioned various vitamins such as vitamin A, $B_1$, $B_2$, $B_6$, $B_{12}$, C, D, E, F, H, K, M and Q, and derivatives thereof; polyols such as glycerol, nitroglycerin, diglycerides and triglycerides, and derivatives thereof; saccharides such as glucose, fructose, galactose, mannose, inositol, maltose, lactose and sucrose, and derivatives thereof; polysaccharides such as hyaluronic acid and chondroitin sulfate, and derivatives thereof; saccharide phosphoric esters such as glucose-1-phosphate, glucose-6-phosphate, mannose-6-phosphate, galactose-6-phosphate, fructose-6-phosphate, glucose-1,6-diphosphate, fructose-1,6-diphosphate and fructose-2,6-diphosphate, alkali metal salts thereof, and derivatives thereof; amino acids such as alanine, leucine, lysine, asparagine, aspartic acid, cysteine, proline, glutamine, serine, glutamic acid, glycine, histidine, tyrosine, isoleucine and valine, and derivatives thereof; sterols such as cholesterol and derivatives thereof; ceramides and derivatives or analogues thereof; polyunsaturated fatty acids such as linolic acid, linolenic acid, arachidonic acid, docosahexaenic acid, prostaglandin, prostacycline and leucotriene, and derivatives thereof; various kinds of hormone and derivatives thereof; peptides and derivatives thereof; porphyrins and derivatives thereof; various kinds of protein such as antibodies and enzymes, and derivatives thereof; ultraviolet absorbents such as 4-tert-butyl-4'-methoxydibenzoylmethane and para-methoxycinnamic esters; blood-circulation accelerators such as octylphthalide; whitening agents such as kojic acid and arbutin; and various kinds of physiologically active substances such as antifungal agents and mildewproofing agents, and derivatives and analogues thereof.

In this invention, no particular limitation is imposed on the concentration of a substance to be encapsulated and encapsulating method thereof. In the case where a hydrophobic substance is encapsulated, however, the proportion of this substance to be incorporated is controlled so as not to destroy the structure of the vesicle.

In order to incorporate the substance to be encapsulated, it is only necessary to dissolve the substance in an aqueous dispersion of a vesicular membrane-forming component containing the phosphoric ester (2) if the substance is soluble in water, or to mix the substance with the membrane-forming component in advance and then disperse the resulting mixture in water if the substance has minimal solubility in water

and strong affinity with the membrane-forming component.

In order to obtain a polymeric vesicle in which an effective ingredient (physiologically active substance or the like) has been encapsulated, it is only necessary to form a vesicle in which the effective ingredient has been encapsulated and polymerize the resulting vesicle. Alternatively, it is possible to first form a vesicle free of any substances to be encapsulated, polymerize the vesicle into a polymeric vesicle and then add a substance to be encapsulated in the polymeric vesicle.

The phosphoric ester (2) which is a membrane-forming ingredient for a vesicle according to this invention has a hydrophilic moiety composed of a betaine structure of a phosphoric acid group and an amino group, and a hydrophobic moiety. Use of phosphoric ester (2) readily permits the industrial formation of a vesicle. Further, since the phosphoric ester (2) has a polymerizable group on the hydrophilic terminus, it is possible to form, by polymerization, a high-molecular weight polymeric vesicle which is physically strong and chemically stable.

Accordingly, the vesicle and polymeric vesicle of this invention have functional features, such as biocompatibility, that vesicles composed of natural phospholipides possess, are stable and strong and can be readily obtained industrially. They are thus valuable as synthetic polymers for use in fields such as engineering and medical science, for example, as various kinds of functional material and microcapsules.

Having generally described this invention, a further understanding can be obtained by reference to certain specific examples which are provided herein for purposes of illustration only and are not intended to be limiting unless otherwise specified.

## EXAMPLES

Reference Example 1:

A reactor was charged with 20.1 g (36.6 mmol) of the sodium salt of 2-decyltetradecyl 2-hydroxy-3-chloropropylphosphate, 118 g of isopropyl alcohol and 9.2 ml of 4N aqueous sodium hydroxide. The contents were stirred at room temperature for 30 minutes. After a solution with 6.2 g (36.7 mmol) of N-(3-dimethylaminopropyl)methacrylamide dissolved in 36.7 ml of 1N aqueous hydrochloric acid was then added dropwise, the contents were heated to 55 ° C to react them for 12 hours. After completion of the reaction, the reaction mixture was diluted with 1000 ml of water and then caused to pass through an electrodialyser to remove ionic impurities. Further, the solvent was distilled off, and the residue was dried under reduced pressure, thereby obtaining 23.0 g (yield: 95.1%) of a phosphoric ester having a structure represented by the following formula (8) as a hygroscopic white solid.

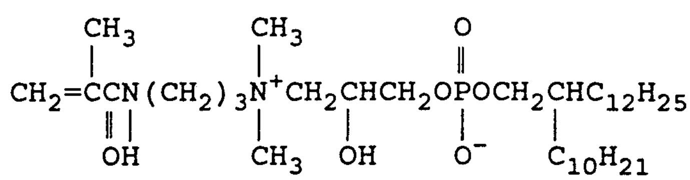

(8)

| | |
|---|---|
| $^1$H-NMR (δ ppm from $CDCl_3$): | 0.05-1.47(m,54H), 1.78(s,3H), 3.03(s,6H), 3.14-3.88(m,7H), 4.23(m,1H), 5.45(d,2H,J = 11.8Hz) |
| $^{13}$C-NMR (δ ppm from $CDCl_3$): | 13.78, 18.41, 22.36, 26.41, 29.06, 29.36, 29.42, 29.82, 30.45, 31.60, 36.31, 38.54, 51.67, 62.65, 64.78, 66.46, 68.17, 119.94, 139.16, 168.92 |
| IR(neat ν; $cm^{-1}$): | 3244, 1659, 1617, 1230, 1068, 654 |
| FAB-MS: | 661$[M+H]^+$ |

Reference Example 2:

A reactor was charged with 20.1 g (30.4 mmol) of the sodium salt of 2-tetradecyloctadecyl 2-hydroxy-3-chloropropylphosphate, 100 g of tetrahydrofuran and 7.6 ml of 4N aqueous sodium hydroxide. The contents were stirred at room temperature for 30 minutes. After a solution with 5.3 g (31.0 mmol) of N-(3-dimethylaminopropyl)methacrylamide dissolved in 31.0 ml of 1N aqueous hydrochloric acid was then added dropwise, the contents were heated to 55 ° C to react them for 10 hours. After completion of the reaction, the reaction mixture was added with 150 g of an amphoteric ion exchange resin and stilled at room temperature for 1 hour. The resin was then separated by filtrations. Further, the solvent was distilled off, and

the residue was dried under reduced pressure, thereby obtaining 11.5 g (yield: 48.0%) of a phosphoric ester having a structure represented by the following formula (9) as a hygroscopic white solid.

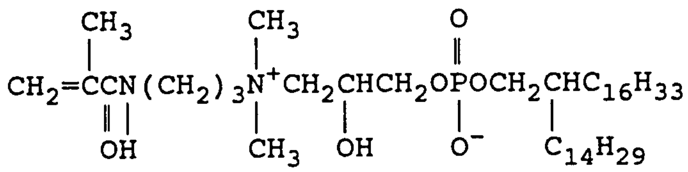

(9)

Example 1:

To 5.00 ml of water were added 50.0 mg of the phosphoric ester (8) prepared in Reference Example 1, 50.0 mg of D-glucose and 5.00 mg of p-methoxyphenol as a polymerization inhibitor, and the resulting mixture was stirred at 50° C for 30 mins. to homogeneously disperse them. The resulting solution was cooled to room temperature, thereby obtaining a turbid vesicular solution. The average diameter of vesicles in this solution was found to be 1540 nm. After the vesicular solution was left over for 2 weeks at room temperature, the average diameter of vesicles in this solution was measured and found to be 1580 nm. The appearance of the solution remained substantially unchanged.

Example 2:

A portion of the vesicular solution prepared in Example 1 was exposed to ultraviolet rays for 3 hours at room temperature under a nitrogen stream to polymerize the vesicles, and the resulting polymerization mixture was observed through an optical microscope. As a result, it was confirmed that polymeric vesicles were present as spherical particles having an average diameter of 1700 nm.

Comparative Example 1:

After 60 mg of dipalmitoylphosphatidyl choline was mixed with 6.00 ml of an aqueous solution containing 0.056 M D-glucose and 0.01 M of ammonium sulfate, the resulting mixture was stirred for 30 minutes while heating it at 50° C. After confirming the formation of a homogeneous dispersion, the dispersion was cooled to room temperature under stirring to obtain a turbid solution of liposome. This solution was subjected to gel filtration on a Sephadex-G50 medium (manufactured by Pharmacia AB) using 0.01 M aqueous solution of ammonium sulfate as an eluent. Since the particle size distribution of the resultant liposome was wide, the fraction containing the liposome extended over a wide range. When the solution before subjected to the gel filtration was left at rest at room temperature for 1 hour, white precipitate was produced. It was hence understood that the liposome formed was unstable. When this precipitate was observed through an optical micrometer, particles of various sizes ranging from 1000 nm to 5000 nm in diameter could be viewed.

Example 3:

A portion of the vesicular solution prepared in Example 1 was subjected to ultrasonic irradiation (100 W/1 minute) using a tip type ultrasound irradiation apparatus at room temperature. After the irradiation, a slightly turbid solution of vesicles was obtained. The average diameter of the vesicles in this solution was found to be 67 nm. The resulting vesicular solution was subjected to gel filtration on a Sephadex-G50 medium (manufactured by Pharmacia AB) using water as an eluent to separate the solution into a vesicle-containing fraction and a fraction containing D-glucose which had not been held by the vesicles. With respect to the vesicle-containing fraction, the concentration of D-glucose and the content of the phosphoric ester were determined. As a result, the trap volume of the vesicles was found to be 1.30 l/mol as expressed in terms of volume per mol of the phosphoric ester.

Example 4:

To 5.00 ml of water were added 50.0 mg of the phosphoric ester (9) prepared in Reference Example 2

and 50.0 mg of D-glucose, and the resulting mixture was stirred at 50°C for 30 mins.to homogeneously disperse them. The resulting solution was cooled to room temperature and subjected to ultrasonic irradiation in the same manner as in Example 3, thereby obtaining a slightly turbid solution of vesicles. The average diameter of the vesicles in this solution was found to be 71 nm. This solution was exposed to ultraviolet rays for 3 hours at 0°C under a nitrogen stream to polymerize the vesicles. After completion of the polymerization, a portion of the polymerization mixture was subjected to gel filtration in the same manner as in Example 3. Water was distilled off from a portion of the resultant polymeric vesicle-containing fraction, and the resulting residue was further dried under reduced pressure. Gold was then sputtered on the residue to observe it through a scanning electron microscope. As a result, it was confirmed that the polymeric vesicles retained a spherical structure and had an average diameter of 79 nm. On the other hand, another portion of the polymeric vesicle-containing fraction obtained by the gel filtration was left over for 2 weeks at room temperature and then subjected to the gel filtration again. As a result, D-glucose isolated from the polymeric vesicles was not detected.

Comparative Example 2:

After 50.0 mg of dipalmitoylphosphatidyl choline was mixed with 5.00 ml of 0.056 M aqueous solution of D-glucose, the resulting mixture was stirred for 30 minutes while heating it at 50°C. After forming a homogeneous dispersion, the dispersion was cooled to room temperature and then subjected to ultrasonic irradiation using a tip type ultrasound irradiation apparatus in the same manner as in Example 3, thereby obtaining a substantially transparent solution of liposome. This solution was subjected to gel filtration using water as an eluent in the same manner as in Example 3, thereby separating it into a liposome-containing fraction and a fraction containing isolated D-glucose which had not been encapsulated in the liposome. The average diameter of the liposome in the resulting liposome-containing fraction was found to be 65 nm. When this solution was left over for 2 weeks at room temperature, the solution became opaque and precipitate was produced. It was hence understood that the liposome formed was poor in stability. When this precipitate was observed through an optical micrometer, particles of various sizes ranging from 1000 nm to 5000 nm in diameter were viewed.

Example 5:

A reactor was charged with 50.0 mg of a phosphoric ester represented by the following formula (10):

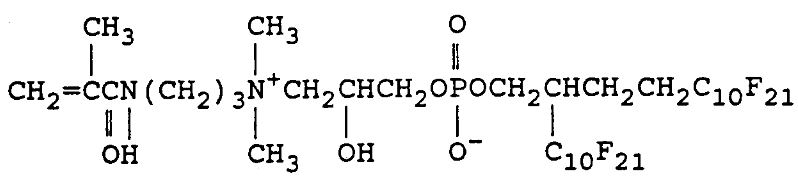

(10)

50.0 mg of the disodium salt of glucose-1-phosphate and 5.00 ml of water. The contents were stirred at 50°C for 30 minutes to homogeneously disperse them. The resulting solution was cooled to room temperature and then subjected to ultrasonic irradiation in the same manner as in Example 3. After the ultrasonic irradiation, the average diameter of the resulting vesicles in the solution was measured and found to be 138 nm. After this solution was exposed to ultraviolet rays for 3 hours at 20°C under a nitrogen stream to polymerize the vesicles, the polymerization mixture was subjected to gel filtration in the same manner as in Example 3, thereby obtaining a polymeric vesicle-containing fraction. Water was distilled off from this fraction, and the resulting residue was further dried under reduced pressure. Gold was then sputtered on the residue to observe it through a scanning electron microscope. As a result, it was confirmed that the polymeric vesicles retained a spherical structure and had an average diameter of 146 nm.

Example 6:

To 100 ml water were added 1.00 g of the phosphoric ester (9) prepared in Reference Example 2 and 100 mg of hexadecyltrimethylammonium bromide. The contents were stirred at 50°C for 30 minutes to homogeneously disperse them, thereby obtaining a turbid vesicular solution. The average diameter of vesicles in this solution was found to be 1480 nm. After this solution was subjected to ultrasonic irradiation

in the same manner as in Example 3, the average diameter of the resulting vesicles contained in the solution was measured and found to be 160 nm. This solution was then exposed to ultraviolet rays for 3 hours at 20°C under a nitrogen stream to polymerize the vesicles. The average diameter of polymeric vesicles obtained after the irradiation was found to be 203 nm.

Example 7:

In 100 ml water were placed 1.00 g of the phosphoric ester (9) prepared in Reference Example 2, 180 mg sodium dihexadecylphosphate and 1.20 g of glycine. The contents were stirred at 50°C for 30 minutes to homogeneously disperse them. The average diameter of vesicles in the resulting solution was measured and found to be 1240 nm. When this solution was subjected to ultrasonic irradiation in the same manner as in Example 3, the solution became substantially transparent. The average diameter of vesicles in this solution was found to be 96 nm. This solution was subjected to gel filtration in the same manner as in Example 3, thereby obtaining a vesicle-containing fraction. This fraction was exposed to ultraviolet rays for 6 hours at 30°C under a nitrogen stream to polymerize the vesicles. The particle size of polymeric vesicles obtained after the irradiation was measured and found to be 94 nm in terms of their average diameter.

Example 8:

After 50.0 mg of the phosphoric ester (8) prepared in Reference Example 1 and 1.50 mg of cholesterol were dissolved in 5.00 ml of chloroform, the solvent was distilled off in a rotary evaporator. Further, the residue was dried under reduced pressure to form a thin membrane. Next, 62.0 mg of D-glucose and 5.00 ml of water were added, and the mixture was then shaken in a vortex mixer to prepare a vesicular solution. The average diameter of vesicles in this solution was found to be 2180 nm. This solution was then subjected to ultrasonic irradiation in the same manner as in Example 3. As a result, the solution became substantially transparent. The average diameter of particles in this solution was measured and found to be 89 nm. This solution was then exposed to ultraviolet rays for 3 hours at 30°C under a nitrogen stream. After the irradiation, the solution was subjected to gel filtration to obtain a polymeric vesicle-containing fraction. The average diameter of polymeric vesicles in this fraction was then measured and found to be 85 nm.

Example 9:

After 1.00 g of the phosphoric ester (9) prepared in Reference Example 2 and 115 mg of dodecyl methacrylate were dissolved in 10.0 ml of chloroform, the solvent was distilled off in a rotary evaporator, and the residue was dried under reduced pressure to form a thin membrane. Next, 20.0 ml of water was added, and the mixture was then shaken in a vortex mixer to prepare a vesicular solution. The average diameter of particles in this solution was found to be 1160 nm. This solution was subjected twice to French press under a pressure of 1500 kg/cm$^2$ at room temperature, thereby obtaining a solution which contained vesicles having an average diameter of 148 nm. After this solution was exposed to ultraviolet rays for 3 hours at 30°C to polymerize the vesicles, water was distilled off from this solution, and the residue was further dried under reduced pressure, thereby obtaining polymeric vesicles. The polymeric vesicles thus obtained were poured in 2.00 ml of water with 100 mg of glycerol dissolved therein, and the mixture was stirred at room temperature for 60 minutes. The resulting solution was subjected to gel filtration in the same manner as in Example 3, thereby collecting a polymeric vesicle-containing fraction. The average diameter of polymeric vesicles contained in this solution was found to be 159 nm.

Example 10:

After 100 g of the same phosphoric ester (10) as used in Example 5 and 5.0 mg of 2-hydroxyethyl methacrylate were dissolved in 2.00 ml of chloroform, the solvent was distilled off in a rotary evaporator, and the residue was dried under reduced pressure to form a thin membrane. Next, 10.0 ml of water was added, and the mixture was then shaken in a vortex mixer to prepare a vesicular solution. The average diameter of particles in this solution was found to be 1230 nm. This solution was subjected twice to French press under a pressure of 1500 kg/cm$^2$ at room temperature, thereby obtaining a solution which contained vesicles having an average diameter of 147 nm. After this solution was exposed to ultraviolet rays for 3 hours at 30°C to polymerize the vesicles, water was distilled off from this solution, and the residue was further dried under reduced pressure, thereby obtaining polymeric vesicles. The polymeric vesicles thus obtained were poured in 0.50 ml of water with 20 mg of D-glucose dissolved therein, and the mixture was

stirred at room temperature for 60 minutes. The resulting solution was subjected to gel filtration in the same manner as in Example 3, thereby collecting a polymeric vesicle-containing fraction. The average diameter of polymeric vesicles contained in this solution was found to be 157 nm.

Example 11:

After 1.00 g of the phosphoric ester (8) prepared in Reference Example 1, 85 mg of dimethylaminoethyl methacrylate and 110 mg of dipalmitoylphosphatidyl choline were dissolved in 10.0 ml of chloroform, the solvent was distilled off in a rotary evaporator, and the residue was dried under reduced pressure to form a thin membrane. Next, 40.0 ml of water was added, and the mixture was then shaken in a vortex mixer to prepare a vesicular solution. The average diameter of particles in this solution was found to be 1500 nm. This solution was subjected to ultrasonic irradiation in the same manner as in Example 3, thereby obtaining a solution which contained vesicles having an average diameter of 138 nm. This solution was then exposed to ultraviolet rays for 3 hours at room temperature under a nitrogen stream to polymerize the vesicles. After water was then distilled off from this solution, and the residue was further dried under reduced pressure, the resulting solid was poured in 1.00 ml of water with 20 mg of glycerol dissolved therein, and the mixture was stirred at room temperature for 60 minutes. The resulting solution was subjected to gel filtration in the same manner as in Example 3, thereby collecting a polymeric vesicle-containing fraction. The average diameter of polymeric vesicles contained in this solution was found to be 120 nm.

Example 12:

In 40.0 ml water were placed 1.00 g of the phosphoric ester (9) prepared in Reference Example 2, 50.0 mg of cholesterol and 120 mg of methyl methacrylate. The contents were stirred at 30 °C for 1 hour to homogeneously disperse them. The average diameter of particles contained in this solution was then measured and found to be 1760 nm. This solution was subjected to ultrasonic irradiation in the same manner as in Example 3. As a result, the solution became substantially transparent, and a solution which contained vesicles having an average diameter of 88 nm was obtained. This solution was then exposed to ultraviolet rays for 3 hours at room temperature under a nitrogen stream to polymerize the vesicles. After the irradiation, water was distilled off from this solution, and the residue was further dried under reduced pressure. The resulting polymeric vesicles were poured in 2.00 ml of water with 100 mg of glycerol dissolved therein, and the mixture was stirred at room temperature for 60 minutes. The resulting solution was subjected to gel filtration in the same manner as in Example 3, thereby collecting a polymeric vesicle-containing fraction. The average diameter of polymeric vesicles contained in this solution was found to be 109 nm.

Obviously, numerous modifications and variations of the present invention are possible in light of the above teachings. It is therefore to be understood that within the scope of the appended claims, the invention may be practiced otherwise than as specifically described herein.

## Claims

**1.** A vesicle comprising, as a membrane-forming ingredient, a phosphoric ester represented by the following general formula (2):

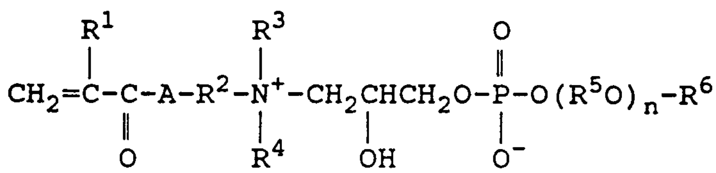

(2)

wherein $R^1$ means a hydrogen atom or an alkyl group having 1-4 carbon atoms, $R^2$ denotes an alkylene group having 1-6 carbon atoms, $R^3$ and $R^4$ may be identical to or different from each other and represent individually an alkyl group having 1-4 carbon atoms, $R^5$ stands for an alkylene group having 2-3 carbon atoms, $R^6$ means a linear or branched alkyl or alkenyl group which may be substituted by halogen atoms and has 8-36 carbon atoms, a phenyl group which has been substituted by a linear or branched alkyl group having 3-15 carbon atoms or a group represented by the following general

formula (3) or (4):

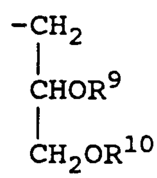
(3)

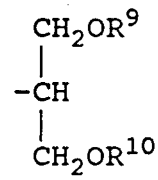
(4)

in which $R^9$ and $R^{10}$ may be identical to or different from each other and mean individually a hydrogen atom, a saturated or unsaturated, linear or branched acyl group having 5-36 carbon atoms or a linear or branched alkyl or alkenyl group having 5-36 carbon atoms, provided that both $R^9$ and $R^{10}$ are not a hydrogen atom at the same time, A denotes NH or an oxygen atom, and n stands for a number of from 0-30.

2. A vesicle according to claim 1, wherein $R^6$ in the general formula (2) is a branched alkyl or alkenyl group which may be substituted by halogen atoms and has 8-36 carbon atoms.

3. A vesicle according to claim 1, further comprising, as a membrane-forming ingredient, at least one component selected from surfactants, unsaturated monomers other than the phosphoric ester (2)-,membrane-stabilizing agents and antioxidants in addition to the phosphoric ester represented by the general formula (2).

4. A vesicle according to claim 1, wherein a physiologically active substance is encapsulated therein.

5. A polymeric vesicle comprising, as a membrane-forming ingredient, a polymer of a phosphoric ester represented by the following general formula (2):

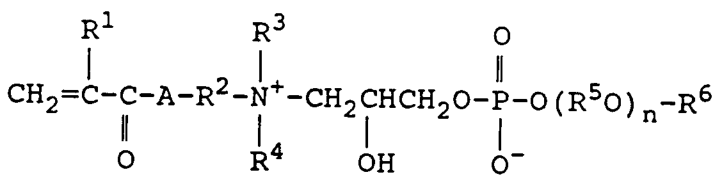
(2)

wherein $R^1$ means a hydrogen atom or an alkyl group having 1-4 carbon atoms, $R^2$ denotes an alkylene group having 1-6 carbon atoms, $R^3$ and $R^4$ may be identical to or different from each other and represent individually an alkyl group having 1-4 carbon atoms, $R^5$ stands for an alkylene group having 2-3 carbon atoms, $R^6$ means a linear or branched alkyl or alkenyl group which may be substituted by halogen atoms and has 8-36 carbon atoms, a phenyl group which has been substituted by a linear or branched alkyl group having 3-15 carbon atoms or a group represented by the following general formula (3) or (4):

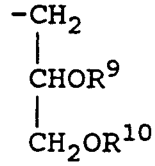
(3)

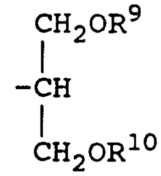
(4)

in which $R^9$ and $R^{10}$ may be identical to or different from each other and mean individually a hydrogen atom, a saturated or unsaturated, linear or branched acyl group having 5-36 carbon atoms or a linear or branched alkyl or alkenyl group having 5-36 carbon atoms, provided that both $R^9$ and $R^{10}$ are not a hydrogen atom at the same time, A denotes NH or an oxygen atom, and n stands for a number of from 0-30.

**6.** A polymeric vesicle according to claim 5, wherein $R^6$ in the general formula (2) is a branched alkyl or alkenyl group which may be substituted by halogen atoms and has 8-36 carbon atoms.

**7.** A polymeric vesicle according to claim 5, further comprising, as a membrane-forming ingredient, at least one component selected from the group consisting of (a) surfactants, (b) unsaturated monomers other than the phosphoric ester (2), (c) polymers obtained from (b), (d) copolymers of the phosphoric ester (2) and monomers (b), (e) membrane-stabilizing agents, and (f) antioxidants.

**8.** A polymeric vesicle according to claim 5, wherein a physiologically active substance is encapsulated therein.

European Patent Office

# EUROPEAN SEARCH REPORT

Application Number

EP 92 10 3902

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,X | PATENT ABSTRACTS OF JAPAN vol. 12, no. 128 (C-489)20 April 1988 & JP-A-62 249 994 ( KAO CORP ) 30 October 1987 | 1,2,4-6, 8 | A61K9/127 |
| Y | * abstract * | 3,7 | |
| X | EP-A-0 036 155 (MAX-PLANCK-GESELLSCHAFT ZUR FÖRDERUNG DER WISSENSCHAFTEN E.V.) * page 2, line 1 - page 9, line 15 * * page 10, line 16 - page 13, line 19 * | 1,2,4-6, 8 | |
| Y | EP-A-0 324 455 (RIBI) *the abstract; col. 4,line 3- col.14, line 23* | 3,7 | |
| A | EP-A-0 395 382 (NIPPON OIL AND FATS COMPANY) * page 1, line 1 - page 5, line 20 * | 1-8 | |
| Y | FR-A-2 579 891 (INTERNATIONAL GENETIC SCIENCES PARTNERSHIP) * claims 1-11 * | 3,7 | |
| A | EP-A-0 160 266 (TERUMO KABUSHIKI KAISHA) * the whole document * | 3,6 | TECHNICAL FIELDS SEARCHED (Int. Cl.5 ) |
| | | | A61K |

The present search report has been drawn up for all claims

| Place of search | Date of completion of the search | Examiner |
|---|---|---|
| THE HAGUE | 24 JUNE 1992 | BENZ K.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)